# EUROPEAN PATENT SPECIFICATION

(11) **EP 1 994 387 B1**
(45) Date of publication and mention of the grant of the patent: **21.03.2012**
(21) Application number: 07717619.6
(22) Date of filing: 15.02.2007
(51) Int. Cl.: G01L 9/00, G01L 19/00

(54) **Disposable pressure sensor and a method of forming it**
Einweg-Drucksensor und Verfahren zur dessen Herstellung
Capteur de pression à usage unique et procédé de production de celui-ci

(30) Priority: 17.02.2006 US 357497
(43) Date of publication of application: 26.11.2008
(73) Proprietor: Honeywell International Inc., Morristown, NJ 07962 (US)
(72) Inventor: BRADLEY, Alistair, D., Dublin, OH 43016 (US); SHIFFER, Stephen, R., P.O. Boy 22445 Morristown NJ 07962-2245 (US)
(74) Representative: Buckley, Guy Julian
(86) International application number: PCT/US2007/062170
(87) International publication number: WO 2007/095597

(56) References cited:
- EP-A1- 0 180 662
- EP-A1- 0 522 567
- US-A- 4 539 998

## Description

### TECHNICAL FIELD

Embodiments are generally related to sensors and, more particularly, to pressure sensors and methods of manufacturing such sensors. Embodiments are additionally related to disposable pressure sensors and to disposable pressure sensor systems for use in medical and other applications. Embodiments are also related to disposable pressure assemblies for use with disposable fluid carrying modules, such as fluid cartridges and tubing used in medical applications.

### BACKGROUND

In single-use type applications, such as for example medical systems and instrumentation where re-use of sensors is unfavorable due to cleaning and sterilization requirements, disposable sensors are required which can be implemented in a cost-effective manner.

Electronic packages for sensors generally have a base level of packaging for housing the sensor followed by further levels of packaging for housing electrical and mechanical connections which are required so that the sensor can be properly interface with the device. Typical sensor assemblies/packages are therefore not particularly well suited to such applications by virtue of the relatively high number of component parts, expensive materials and/or processing requirements, and high number of manufacturing-processing steps required to both produce packaged sensors and to integrate them into the instrumentation or apparatus of the application. EP 0 180 662 A1 discloses a disposable measuring transducer for medical applications. US 4 539 998 shows a pressure transducer assembly and EP 0 522 567 A1 shows a pressure converter.

Pressure sensing solutions, particularly for disposable sensors, are therefore required to provide an ultra low cost assembly which can be integrated more easily and more cost effectively into the final application or system.

The embodiments disclosed herein therefore directly address the shortcomings of present pressure sensors providing low cost disposable pressure sensor assemblies and packaging associated therewith which can be integrated into instrumentation and other equipment simply and cost effectively.

### BRIEF SUMMARY

The present invention provides a disposable packaged pressure sensor as defined in Claim 1.

The sensor may include the features of any one or more of dependent Claims 2 to 13.

The present invention also provides a method of forming a disposable packaged pressure sensor as defined in Claim 14.

The method may include the features of any one or more of dependent Claims 15 and 16.

The following summary is provided to facilitate an understanding of some of the innovative features unique to the present invention and is not intended to be a full description. A full appreciation of the various aspects of the invention can be gained by taking the entire specification, claims, drawings, and abstract as a whole.

Disposable pressure sensors can be used in medical applications, such as blood pressure sensing.

The aforementioned aspects and other objectives and advantages can now be achieved as described herein. A disposable package for a pressure sensor is disclosed. The package has a housing or frame for carrying at least one sensing element thereon and at least one mechanical and/or electrical connector for connecting the sensing element(s) to an external apparatus. The one or more connectors are integrated in the housing so that both the connector(s) and sensing element(s) are packaged in a single part.

By incorporating the sensing element(s), such as pressure sensor die(s), on the same frame or housing as the electrical connector, many of the parts and electrical connections necessary to make the sensor are eliminated. For example, a separate dedicated housing is no longer required to accommodate the sensor. Furthermore, incorporating the electrical connector on the frame eliminates the need to provide a separate cable connector assembly for electrically connecting the sensor to a cooperating connector of the external apparatus. Also, a plurality of sensing elements can be attached to the same housing. Consequently, the sensor system can be manufactured with less parts and associated processing steps thereby enabling a low cost sensor system to be provided.

The one or more mechanical connectors can be in the form of mechanical connection(s) integrally formed in the housing or frame for connecting the sensor to a fluid carrying module, such as a dialysis cartridge. Alternatively or additionally, the housing can include integrally formed mechanical and/or electrical connections for connecting the sensor(s) to a measurement apparatus for measuring the output signals of the sensor(s). The disposable package can include one or more windows or ports, also integrally formed in the housing, for transmitting a fluid pressure to the sensing element(s) which can be, for example, pressure sensing elements. One or more sealing interface surfaces or connectors for sealing the housing window(s) or port(s) to corresponding fluid ports of a device can also be integrated in the housing.

The housing or frame can be a molded plastic part having a patterned metalized layer so as to form the electrical connections required to incorporate the sensor into the application.

According to another aspect a disposable pressure sensor system has a disposable sensor assembly which has one or more sensing elements carried on a housing or frame. One or more electrical and/or mechanical connectors for connecting the sensing element(s) to an apparatus or device are integrated in the housing. Also integrated in the housing, can be a window or port for transmitting a fluid pressure to the sensing element(s). One or more sealing connections can be integrated in the housing for sealing the housing to a device such that the sensing element(s) can detect pressure of fluid held or flowing through the device.

One or more of the mechanical connectors can be a mechanical connection for securely attaching the pressure sensor assembly to a fluid carrying module and/or measuring apparatus.

The disposable pressure sensor system can include a fluid carrying module, such as a cartridge for a dialysis machine, and one or more of the mechanical connectors integrated in the housing can be a mechanical connection configured to cooperate with a corresponding connection on the fluid carrying module. For example, the mechanical connections of a cartridge and the housing can be configured such that the pressure sensor assembly is snapable or latchable to the cartridge.

If necessary, the housing or frame can be integrated with the fluid carrying module. The fluid carrying module can be, for example, a catheter tube or a dialysis cartridge. One or more trim components can be carried on the housing and electrically connected to the sensing element(s) for adjusting or offsetting an output signal of the sensing element(s). The sensing element(s) can be reactive ion etched diaphragm(s) further reducing the pressure sensor system cost.

According to yet another aspect, a method of forming a disposable pressure sensor system comprises forming a housing or frame, integrally forming a connecting portion in the frame, depositing conductive material on the connecting portion so as to integrate at least one electrical connector in the housing, attaching at least one pressure sensing element on the housing, and electrical connecting sensing elements to the conductive material.

### BRIEF DESCRIPTION OF THE DRAWINGS

The accompanying figures, in which like reference numerals refer to identical or functionally-similar elements throughout the separate views and which are incorporated in and form a part of the specification, further illustrate the present invention and, together with the detailed description of the invention, serve to explain the principles of the present invention.

FIG. 1 illustrates a cross-sectional view of pressure sensor system having a first embodiment of a disposable pressure sensor assembly attached to a disposable fluid carrying module,

FIG. 2 illustrates a plan view taken from the rear side of the pressure sensor assembly shown in FIG. 1 but with one of the protective covers for covering one of the pressure sensing elements removed,

FIG. 3 illustrates a plan view taken from above of a pressure sensor system having a second embodiment of a disposable pressure sensor assembly, aligned with a disposable fluid carrying module, for attachment thereto,

FIG. 4 illustrates a cross-sectional view of a pressure sensor system having another embodiment of a disposable pressure sensor assembly partially inserted into a disposable fluid carrying module,

FIG. 5, illustrates a front perspective view taken from above the assembly of FIG. 4 with the pressure sensor assembly fully inserted into the fluid carrying module, and

FIG. 6 illustrates a cross-sectional view of the pressure sensor assembly of FIG. 4 snap fitted to an external controller with the fluid carrying module carried on the pressure sensor assembly, and

FIGS. 7 & 8 illustrate perspective and exploded views of a disposable medical pressure sensor system according to yet another embodiment.

### DETAILED DESCRIPTION OF THE INVENTION

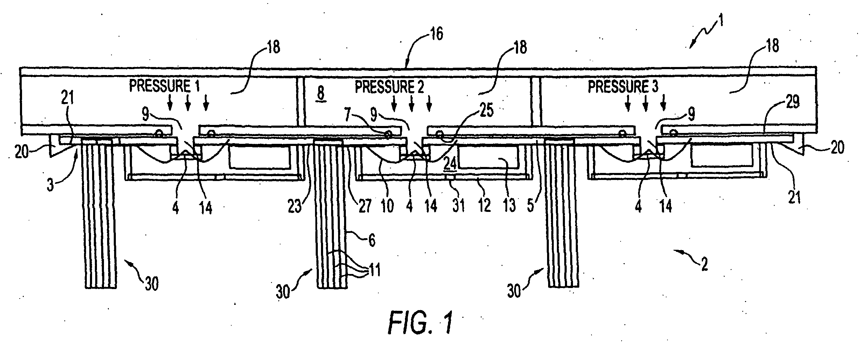

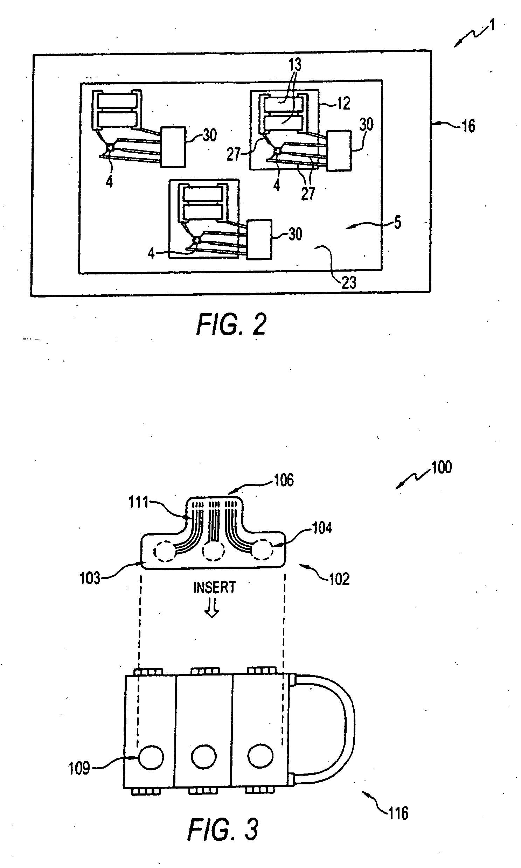

Referring to FIGS. 1 and 2 of the accompanying drawings, which illustrate cross-sectional and rear views of pressure sensor system having a first embodiment of a pressure sensor assembly attached to a fluid carrying module, the pressure sensor system 1 has a pressure sensor assembly or package 2 which includes one or more pressure sensing elements 4 for sensing pressure of fluid 8 held in a disposable fluid carrying module 16, such as a disposable cartridge used in a haemodialysis machine. As will be explained in more detail below, the pressure sensor assembly 2 includes a housing or frame 3 which not only serves as a substrate for the sensing elements 4 but also forms all the electrical and mechanical connections required to incorporate the sensors into the application. Consequently, the sensing elements and their associated connections can be packaged together on one part, that is, the housing 3, which enables a reduction in both material and assembly costs at all stages of manufacture and use.

In the embodiment shown in FIG. 1, housing 3 is formed from plastic, for example injection molded polymer, such as polycarbonate material, but the housing can be made from other suitable materials. Also, in the illustrative embodiment of FIG. 1, the pressure sensor assembly 2 has three pressure sensing elements 4 for sensing pressure of fluid held in three respective individual chambers 18 of module 16, however, the assembly 2 could have a single sensing element 4 or any number of sensing elements 4 for sensing fluid pressure held in any number of chambers 18. Furthermore, the pressure sensor assembly 2 could be used to sense fluid pressure carried by apparatus other than disposable modules 16.

Housing 3 includes a panel 5 which serves as a supporting substrate for sensing elements 4 attached to the rear face 23 of the panel by means of a suitable bonding material, such as room temperature vulcanizing Silicone (RTV) .Sensing elements 4 are arranged to be in communication with corresponding windows or inlets 14, formed in the panel 5, for transmitting fluid under pressure so as to expose one side of the sensing element(s) to the fluid 8 and the other side to a reference pressure 24, for example atmospheric pressure.

Panel 5 also includes mechanical connections 21 for securably attaching the pressure sensor assembly 2 to the module 16 in an operating position in which inlets 14 are contiguous with corresponding outlets 9 of the module such that the sensing elements can detect fluid 8 transmitted thereto from respective chambers 18. Sealing interface surfaces 25 formed on the panel 3 have respective 'O' rings 7 seated therein for sealing the inlets 14 to the corresponding outlets 9.

In this particular embodiment, the peripheral edge of the panel 5 forms the mechanical connection 21 which is latchable onto the module 16 by means of a pair of latching arms 20 extending perpendicularly from opposite sides of the module rear face 29. However, the pressure sensor assembly 2 can be connected to the module 16 using other techniques, such as for example by ultrasonic or laser welding or bonding the mechanical connections 21 to the module. Furthermore, structure other than or in addition to sealing interface surfaces 25 can be incorporated into the housing for sealing the inlets 14 to the outlets 9, such as for example flanges, threaded ports or clips.

Circuitry of the sensing elements 4 are electrically connected by means of wire bonding 10 to metalized plastic contact pads (not shown) which, in turn are connected to metalized plastic electrical interconnects or traces 27. Housing 3 also includes arms 6, extending perpendicularly from panel rear 23, having electrical terminals 11 carried thereon so as to define an electrical connector 30. Electrical terminals 11, electrically coupled to wire bonding 10 by means of the electrical interconnects 27, are arranged for electrical connection to corresponding mating connections of a measurement apparatus (not shown) to which pressure sensor assembly 2 is attachable. Mechanically attaching the pressure sensor assembly 2 to the measurement apparatus causes the electrical terminals 11 to mate with the corresponding mating connections of the measurement apparatus such that outputs signals of the sensing elements 4 can be provided to the measurement apparatus for signal conditioning and processing.

In this particular embodiment, electrical terminals 11 and interconnects 27 are formed by deposition of conductive material onto the housing 3, for example using metal deposition processes known in the art, such as Exact^{™} process developed by Cybershield, or alternatively, direct write processes such as inkjet for deposition of metals and conducting polymers/composites. Deposition of conductive materials could also be used to create ground plain metallization and other similar methods of screening radio-frequency interference from the sensors.

Electrical connectors 30 shown in FIG. 1 are of a rectangular card configuration, however, electrical connectors 30 can be fabricated in any desired shape or format by appropriate molding of the plastic housing 3 and formation of circuit traces or terminals 11. Advantageously, circuit traces 27 can run continuously from the sensors' wire bondings 10 or other sensors' interconnects to any shape of electrical connector 30 without the need for any additional assembly.

The sensing elements 4 shown in the illustrative embodiment of FIG. 1 are micro machined piezoresistive (PZR) silicon devices. Preferred embodiment uses die containing a cavity having vertical side walls formed on a die by reactive ion etching a diaphragm (DRIE) in silicon. Since the cavity has vertical side walls, a smaller die size can be achieved than by standard anisotropic wet etching. In this way the number of die on a 6" wafer can be as high as 30,000, which can lead to a reduced unit die cost. For example die with area (footprint) of just 650umx650um are commercially available. Alternatively other MEMS pressure sensing elements, particularly surface micro machined structures which enable small dimensions due to fabrication of thin diaphragms could be employed.

Each PZR-Si sensing element 4 shown in FIG. 1 is arranged as a Wheatstone bridge on the die ("bare die"). In order to meet accuracy and sensitivity and offset requirements for the application additional trimming and signal conditioning can be provided using trim components as is known in the art. In the illustrative embodiment of FIG. 1, such trimming for each sensing element 4 is provided by associated resistors 13, such as resistors surface mounted on the panel rear 23, electrical connected in series with the bridge by means of electrical interconnects 29 to provide a span trim. For example, one surface mount resistor 13 can be added in parallel after production testing and the other resistor fitted prior to testing so as to enable calibration test measurements using only the four connections used in the application (+/- supply and +/- output). As an example, the resulting series resistance may be provided to set pressure span to 100mV output at 5V supply for 1 bar g. Further trim components can be employed to provide offset correction in the application using a calibration at a reference pressure such as ambient (0 bar gage).

Alternatively, resistors 13 can be replaced with a single thick film resistor whose resistance can be modified by exposure to a laser (i.e. a laser trimmable resistor).

External trim components, such as resistors 13, can be omitted if on-chip trimming is achieved using, for example, chrome-silicon (CrSi) resistors deposited directly on the silicon pressure die in order to allow fewer component parts and lower overall sensor cost. Such on-chip trimming processing can be performed for example by laser trimming the sensor die while in wafer form or after attachment of the sensing element to the housing, thereby allowing for compensation of packaging induced offsets.

A protective cover 12 or cap, clipped or bonded to the panel rear over the sensing elements 4 and circuitry, can be employed to provide the sensing elements and circuitry mechanical protection. If sensing elements and circuitry require protection from liquid and/or gases or from compounds, particles, impurities associated therewith, such as for example in a steam sterilization process, the cover 12 could be sealed in place, for example using an 'O' ring, RTV, epoxy, or adhesive. Where a reference to a pressure media 24 such as atmosphere is required, as is the case in the illustrative embodiment of FIG. 1, the cover 12 includes a vent or porous material 31, such as PTFE, which forms a gas permeable vent/filter to provide reference to atmospheric pressure whilst preventing attack or ingress of moisture etc. Alternatively, a PTFE coating could be applied to seal the protective cover 12 to the housing thereby making a gas permeable layer.

If required, passivation of circuit traces 11,27, wire bonds 10 and/or sensing elements 4 can be employed to prevent exposure of such parts to liquid/gas in the local environment and/or cleaning fluids. For example, a PTFE (Teflon) coating technique, known in the art, like that offered by GVD corporation, Massachusetts, could be used. Alternatively, gel fill material, such as silicone gel, can be applied as is standard in the art to provide physical and/or electrical isolation of the sensing element and/or electrical connections and/or die attach material from the media and/or reference environment.

Furthermore if isolation from EMI is required, cover 12 could be metallized and, if necessary conductive adhesive can be used to connect this to a common point on the metallization of the plastic housing. Cover 12 can also support a label or even a smart label, such as RFID tag, as required to provide various data/data storage for use with the assembly.

Preferably, for ease of production assembly, the pressure sensor assembly is manufactured utilizing reel-to-reel processing such as disclosed in United States Patent Application Publication No. US2005/013659 A1 of Shiffer et al, published on June 23, 2005 and entitled " Plastic Lead Frames Utilizing Reel-to-Reel Processing" which is incorporated herein by reference. A plastic part or substrate is transported on a carrier for manufacturing of the pressure sensor assembly based on the initial part. A reel-to-reel mechanism permits a plurality of manufacturing operations, such as plastic molding operations to form the housing and circuit configuring operations to form the circuitry, to be implemented upon the initial part to create the final pressure sensor assembly.

Preferably, housing 3 is run through a fully automated assembly line in which the metallization process is followed by attachment of the sensing element (RTV/ epoxy/flip chip bonding), wire bonding 10 or other forms of conductive bonding, such as anisotropic conductive adhesive, to make electrical connection to the sensing elements 4, followed by fitting of components and/or calibration/trim/test, followed by passivation and/or attachment of protective cap 12. Assembled parts are kept in reel form to feed into the assembly line for the whole system e.g. OEM manufacturing line, such as that used for manufacture of disposable cartridges/cassettes where the low cost sensor assembly could then be singulated and clipped and sealed into place.

Referring now to FIG. 3, which illustrates plan view of a pressure sensor system having a second embodiment of a disposable pressure sensor assembly, aligned with a disposable fluid carrying module for attachment thereto, the pressure sensor assembly 102 is similar in construction to the pressure sensor assembly of 2 of the embodiment shown in FIG. 1 save that the housing 103 includes single electrical connector head or plug 106 for electrically connecting all three sensing elements 104 to the instrumentation or equipment for processing output signals from the sensing elements 104. The sensor assembly 102 is securely attachable to module 116 which is of similar configuration to the module 16 of FIG.1. The sensor assembly 102 can be snap fitted, bonded or welded to module 116.

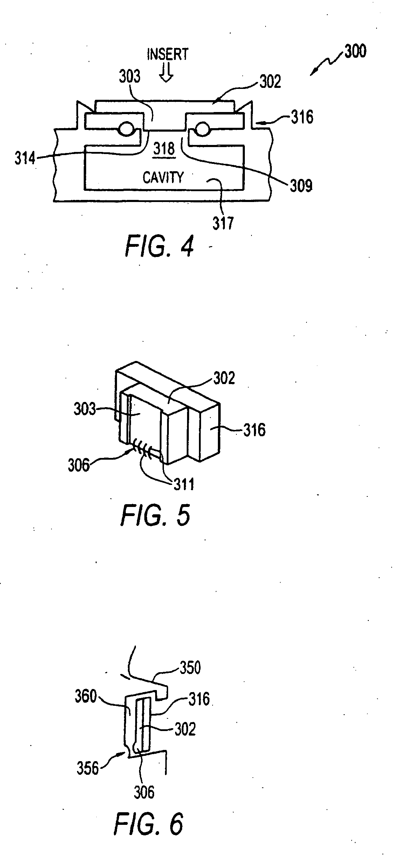

FIGS. 4 and 5 respectively illustrate cross-sectional and perspective views of another embodiment of a passive sensor system having a pressure sensor assembly partially and fully snap fitted to a disposable fluid carrying module 316 which, in this particular embodiment, consists of a disposable cartridge for connection to corresponding permanent/re-usable equipment. Pressure sensor assembly 302 has a similar construction to that of pressure sensor assembly 2 of FIG. 1 with the housing portion shown, 303, having a single inlet or port 314 for transmitting fluid 318 from a cavity 317 in the cartridge 316 to the pressure sensor (not shown). The housing 303 is configured such that port 314 is inserts into a corresponding port 309 of the cartridge as the pressure sensor assembly is snap fitted to the cartridge 316.

As best illustrated in FIG. 5, the electrical connector 306 for electrically connecting the pressure sensor assembly 302 to the permanent equipment consists of a plurality of electrical contact terminals 311 arranged in the rear of the housing. Referring to FIG. 6, which illustrates a cross-sectional side view of the pressure sensor assembly 302, together with the disposable cassette 316 to which the assembly 302 is attached, connected to the permanent equipment 350. The housing 303 of the pressure sensor assembly 302 is configured to be latchable into a cavity 360 of the permanent equipment 350 such that the pressure assembly 302 and associated cassette 316 can be securely attached to the permanent equipment . The permanent equipment 350 includes spring loaded contacts 356, mounted in the cavity 360, which are arranged to make electrical connection with corresponding electrical terminals 311 of the assembly 302 when the pressure assembly is latched in position in the permanent equipment so that output signals from the pressure sensor assembly 302 can be supplied to the permanent equipment for further processing.

The disposable housing 302 and permanent equipment 350 can have alternative mechanical and/or electrical connections to those shown in FIG. 6 for mechanically and/or electrically connecting the pressure assembly, attached to the cassette, to the permanent equipment. Also, one or more channels or passageways and/or ports (not shown) can be molded in the same housing 303, as shown in Fig 3 for example, depending on application requirements for fluid transport/control/measurement.

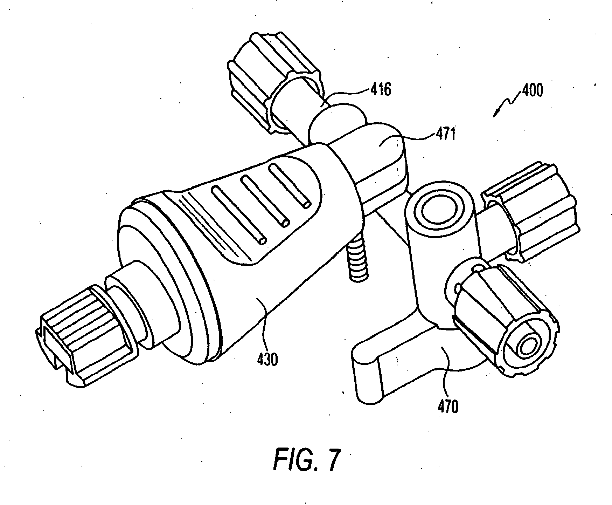

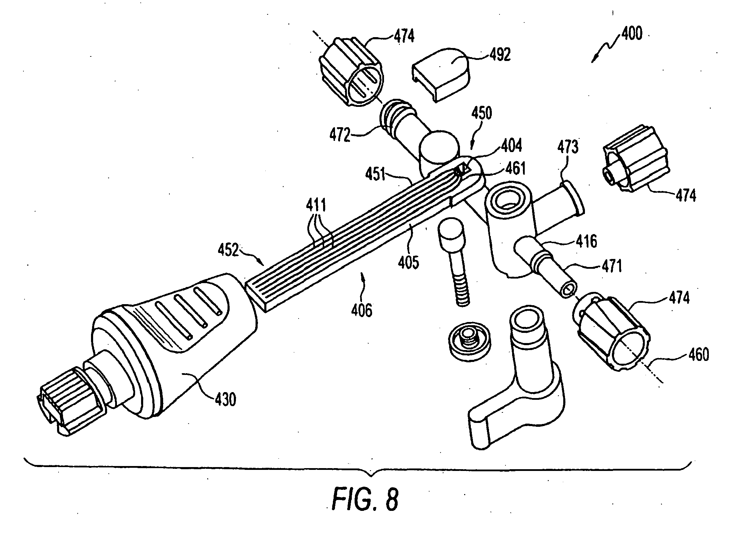

A pressure sensor system according to yet another embodiment is illustrated in FIGS. 7 and 8j which illustrate perspective and exploded views of a disposable medical pressure sensor system. The pressure sensor system 400 is implemented as a disposable catheter 400 for withdrawing fluids from or introducing fluids into the body. One end 450 of the housing or frame 405 of the pressure sensor assembly is integrally formed in the sidewall of the catheter flow tube 416 so as to incorporate the pressure sensor assembly into the flow tube. Frame 405 is of a rectangular configuration extending away from the flow tube in a direction substantially perpendicularly to the flow tube axis 460. A pressure sensor die 404 is mounted directly to the flow tube 416 by locating it on the frame upper side 451 at end 450. The sensor die is sealed over a pressure port or passageway (not shown) communicating_between the sensor die and the interior of the flow tube 416 such that fluid flowing through the flow tube can be applied to the pressure sensor die for sensing thereby. A lid or cap 472 is attachable to the upper side 451, also at end 450, so as to cover and protect the sensor die 404 and sensor interconnects 461.

Electrical connections 411 in the form of conductive traces or interconnects, formed on the frame upper side 451, extend away from the pressure sensor interconnects 461 to the frame distal end 452 which is configured to be insertable into a receiving passageway of an electrical female connector 430 of a measurement apparatus. Frame 405 together with electrical connections 411 define a plastic lead frame 405,411. The frame 405 and electrical connections 411 define an electrical connector 406 for electrical connection with a corresponding connector 430 of a measuring apparatus for measuring the output signals of the pressure sensor.

A stop valve 470 for controlling flow of fluid into the flow tube 416 is also integrally formed in the flow tube as are various push on fittings 471-473 for receiving associated tubing fastened thereon using tube grips or clamps 474.

In the illustrative embodiment of FIG. 7 the fluid carrying part is a catheter flow tube, however, the housing or frame could be integrated into a fluid carrying part other than a flow tube, such as for example the cartridge of the pressure sensor system shown in FIG. 1. Also, the integrated flow tube and frame are formed as a molded plastic unitary structure. However, other materials suitable for forming flow tube and frame as unitary structure capable of carrying fluid and the pressure sensor die and associated circuitry can be employed.

By incorporating the frame 405 into the flow tube 416 or other fluid carrying part, separate mechanical connectors or connections for connecting the pressure assembly to the fluid carrying part, need not be provided. Also by incorporating the pressure sensor die on the plastic lead frame 405,411, many of the parts and electrical connections necessary to make the pressure sensor assembly are eliminated. For example, a separate dedicated housing is no longer required to accommodate the pressure sensor. Furthermore, incorporating the electrical connector on the frame eliminates the need to provide a separate cable connector assembly for electrically connecting the sensor to the female connector. Consequently, the pressure sensor system can be manufactured with less parts and associated processing steps thereby enabling a low cost pressure sensor system to be provided.

The description as set forth is not intended to be exhaustive or to limit the scope of the invention. Many modifications and variations are possible in light of the above teaching without departing from the scope of the following claims. For example, those skilled in the art would understand that the metalized plastic housing having integrated electrical and mechanical connectors, as shown in the illustrative embodiments herein, could be applied to other sensing systems, such as for example temperature, speed and position sensing systems. It is contemplated that the use of the present invention can involve components having different characteristics. It is intended that the scope of the present invention be defined by the claims appended hereto, giving full cognizance to equivalents in all respects.

The embodiments and examples set forth herein are presented to best explain the present invention and its practical application and to thereby enable those skilled in the art to make and utilize the invention. Those skilled in the art, however, will recognize that the foregoing description and examples have been presented for the purpose of illustration and example only. Other variations and modifications of the present invention will be apparent to those of skill in the art, and it is the intent of the appended claims that such variations and modifications be covered.

## Claims

1. A disposable packaged pressure sensor, comprising:
a single substrate (3) comprising a rear face (23) and at least one arm (6) that extends outward from said rear face;
at least one sensing element on said rear face;
electrical interconnects (27) on said rear face;
wire bonds (10) for connecting said sensing element to said electrical interconnects; and
at least one electrical connector (30) on said arm for connecting to said electrical interconnects so that output signals from said at least one sensing element can be coupled to an external apparatus.

2. The disposable packaged pressure sensor of claim 1 wherein said at least one arm (6) extends perpendicular from said rear face (23).

3. The disposable packaged pressure sensor of claim 1 further comprising at least one window or port, integrally formed in said single substrate, for transmitting a fluid pressure to said at least one sensing element.

4. The disposable packaged pressure sensor of claim 3 further comprising at least one sealing interface surface or connector, integrated in said single substrate (3), for sealing said housing window or port to at least one corresponding fluid port of a device.

5. The disposable packaged pressure sensor claim 1 wherein said at least one electrical connector (30) and said electrical interconnects comprises-conductive material deposited onto said single substrate (3).

6. The disposable packaged pressure sensor of claim 1 comprising attachment portions for receiving a plurality of said sensing elements, and electrical interconnects for electrically connecting said sensing elements to respective said electrical connectors (30).

7. The disposable packaged pressure sensor of claim 1 wherein said single substrate (3) comprises a plastic substrate and wherein said plastic substrate is metalized so as to form a plastic lead frame having said at least one electrical connector (30) thereon.

8. The disposable packaged pressure sensor of claim 1 wherein said at least one electrical connector (30) includes electrical terminals formed on said substrate for contacting electrical contacts of a measuring apparatus for measuring the output signals of said at least one sensing element, said electrical terminals being electrically connected to said at least one sensing element.

9. The disposable packaged pressure sensor of claim 1 further comprising a fluid carrying module, said single substrate being formed integrally with said fluid carrying module.

10. The disposable packaged pressure sensor of claim 9 wherein said fluid carrying module comprises a disposable catheter flow tube.

11. The disposable packaged pressure sensor of claim 1 further comprising a disposable cartridge for a dialysis machine and wherein said at least one electrical connector (30) comprises a mechanical connection configured to cooperate with a corresponding connection on said cartridge.

12. The disposable packaged pressure sensor of claim 1 further comprising at least one trim component on said substrate (3) electrically connected to said at least one sensing element for adjusting or offsetting the output signal of said at least one sensing element.

13. The disposable packaged pressure sensor of claim 1 wherein said sensor element comprises a sensor die or chip mounted on said single substrate (3), and further comprising at least one on-chip trim resistor deposited on said sensor die, said on-chip trim resistor being trimable thereby allowing for compensation of span and/or offset.

14. A method of forming a disposable packaged pressure sensor on a single substrate (3) comprising a rear face (23) and at least one arm (6) that extends outward from said rear face of said substrate, said method comprising:
forming electrical interconnects on said rear face;
attaching at least one pressure sensing element on said rear face,
wire bonding to connect said pressure sensing element to said electrical interconnects;
and
forming at least one electrical connector (30) on said arm, wherein said electrical connector provides a connection to said electrical interconnects so that output signals from said at least one pressure sensing element can be coupled to an external apparatus.

15. The method of claim 14 wherein said single substrate (3) comprises a plastic substrate, further comprising forming said plastic substrate using a molding process, wherein said plastic substrate is a molded plastic substrate.

16. The method of claim 15 wherein said forming said electrical interconnects and said forming said electrical connector (30) comprise depositing a conductive material on said plastic substrate.

## Patentansprüche

1. Wegwerfbarer, in einem Gehäuse angeordneter Drucksensor, der umfasst:
ein einzelnes Substrat (3), das eine hintere Fläche (23) und wenigstens einen Arm (6), der sich von der hinteren Fläche nach außen erstreckt, aufweist;
wenigstens ein Erfassungselement auf der hinteren Fläche;
elektrische Verbindungsleitungen (27) auf der hinteren Fläche;
Drahtkontaktierungen (10), um das Erfassungselement mit den elektrischen Verbindungsleitungen zu verbinden; und
wenigstens einen elektrischen Verbinder (30) an dem Arm für den Anschluss an die elektrischen Verbindungsleitungen, so dass Ausgangssignale vom den wenigstens einen Erfassungselement mit einer externen Vorrichtung gekoppelt werden können.

2. Wegwerfbarer, in einem Gehäuse angeordneter Drucksensor nach Anspruch 1, wobei sich der wenigstens eine Arm (6) senkrecht von der hinteren Fläche (23) erstreckt.

3. Wegwerfbarer, in einem Gehäuse angeordneter Drucksensor nach Anspruch 1, der ferner wenigstens ein Fenster oder eine Öffnung aufweist, das bzw. die in dem einzelnen Substrat integral ausgebildet ist, um einen Fluiddruck an das wenigstens eine Erfassungselement zu übertragen.

4. Wegwerfbarer, in einem Gehäuse angeordneter Drucksensor nach Anspruch 3, der ferner wenigstens eine Abdichtungsgrenzfläche oder einen Verbinder umfasst, die bzw. der in das einzelne Substrat (3) integriert sind, um das Gehäusefenster bzw. die Gehäuseöffnung mit wenigstens einer entsprechenden Fluidöffnung einer Vorrichtung dicht zu verbinden.

5. Wegwerfbarer, in einem Gehäuse angeordneter Drucksensor nach Anspruch 1, wobei der wenigstens eine elektrische Verbinder (30) und die elektrischen Verbindungsleitungen leitendes Material enthalten, das auf dem einzelnen Substrat (3) abgelagert ist.

6. Wegwerfbarer, in einem Gehäuse angeordneter Drucksensor nach Anspruch 1, der Befestigungsabschnitte aufweist, um mehrere der Erfassungselemente aufzunehmen, und elektrische Verbindungsleitungen aufweist, um die Erfassungselemente mit entsprechenden elektrischen Verbindern (30) elektrisch zu verbinden.

7. Wegwerfbarer, in einem Gehäuse angeordneter Drucksensor nach Anspruch 1, wobei das einzelne Substrat (3) ein Kunststoffsubstrat umfasst und wobei das Kunststoffsubstrat mit Metall beschichtet ist, um einen Kunststoff-Leiterrahmen zu bilden, an dem sich der wenigstens eine elektrische Verbinder (30) befindet.

8. Wegwerfbarer, in einem Gehäuse angeordneter Drucksensor nach Anspruch 1, wobei der wenigstens eine elektrische Verbinder (30) elektrische Anschlussklemmen aufweist, die an dem Substrat ausgebildet sind, um mit elektrischen Kontakten einer Messvorrichtung zum Messen der Ausgangssignale des wenigstens einen Erfassungselements in Kontakt zu gelangen, wobei die elektrischen Anschlussklemmen mit dem wenigstens einen Erfassungselement elektrisch verbunden sind.

9. Wegwerfbarer, in einem Gehäuse angeordneter Drucksensor nach Anspruch 1, der ferner ein Fluidträgermodul umfasst, wobei das einzelne Substrat einteilig mit dem Fluidträgermodul ausgebildet ist.

10. Wegwerfbarer, in einem Gehäuse angeordneter Drucksensor nach Anspruch 9, wobei das Fluidträgermodul ein wegwerfbares Katheterströmungsrohr umfasst.

11. Wegwerfbarer, in einem Gehäuse angeordneter Drucksensor nach Anspruch 1, der ferner eine wegwerfbare Patrone für eine Dialysemaschine umfasst und wobei der wenigstens eine elektrische Verbinder (30) eine mechanische Verbindungsleitung aufweist, die konfiguriert ist, um mit einer entsprechenden Verbindungsleitung an der Patrone zusammen zu wirken.

12. Wegwerfbarer, in einem Gehäuse angeordneter Drucksensor nach Anspruch 1, der ferner wenigstens eine Abstimmkomponente an dem Substrat (3) aufweist, die mit dem wenigstens einen Erfassungselement elektrisch verbunden ist, um das Ausgangssignal des wenigstens einen Erfassungselements einzustellen oder zu versetzen.

13. Wegwerfbarer, in einem Gehäuse angeordneter Drucksensor nach Anspruch 1, wobei das Sensorelement ein Sensorplättchen oder einen Sensorchip aufweist, das bzw. der auf dem einzelnen Substrat (3) montiert ist, und ferner wenigstens einen Onchip-Abstimmwiderstand aufweist, der auf dem Sensorplättchen abgelagert ist, wobei der Onchip-Abstimmwiderstand abstimmbar ist, um dadurch die Kompensation eines Messbereichs und/oder eines Versatzes zu ermöglichen.

14. Verfahren zum Bilden eines wegwerfbaren, in einem Gehäuse angeordneten Drucksensors auf einem einzelnen Substrat (3), das eine hintere Fläche (23) und wenigstens einen Arm (6), der sich von der hinteren Fläche des Substrats nach außen erstreckt, umfasst, wobei das Verfahren umfasst:
Bilden elektrischer Verbindungsleitungen auf der hinteren Fläche;
Befestigen wenigstens eines Druckerfassungselements auf der hinteren Fläche,
Drahtkontaktieren, um das Druckerfassungselement mit den elektrischen Verbindungsleitungen zu verbinden; und
Bilden wenigstens eines elektrischen Verbinders (30) an dem Arm, wobei der elektrische Verbinder einen Anschluss an die elektrischen Verbindungsleitungen schafft, so dass Ausgangssignale von dem wenigstens einen Druckerfassungselement mit einer externen Vorrichtung gekoppelt werden können.

15. Verfahren nach Anspruch 14, wobei das einzelne Substrat (3) ein Kunststoffsubstrat umfasst, ferner umfassend das Bilden des Kunststoffsubstrats unter Verwendung eines Gießprozesses, wobei das Kunststoffsubstrat ein gegossenes Kunststoffsubstrat ist.

16. Verfahren nach Anspruch 15, wobei das Bilden der elektrischen Verbindungsleitungen und das Bilden des elektrischen Verbinders (30) das Ablagern eines leitenden Materials auf dem Kunststoffsubstrat umfasst.

## Revendications

1. Capteur de pression jetable sous boîtier, comprenant :
un seul substrat (3) comprenant une face arrière (23) et au moins un bras (6) qui s'étend vers l'extérieur depuis ladite surface arrière ;
au moins un élément de détection sur ladite face arrière ;
des interconnexions électriques (27) sur ladite face arrière ;
des soudures de fils (10) pour connecter ledit élément de détection auxdites interconnexions électriques ; et
au moins un connecteur électrique (30) sur ledit bras pour faire la connexion auxdites connexions électriques de manière à ce que les signaux de sortie venant dudit au moins un élément de détection puissent être couplés à un appareil externe.

2. Capteur de pression jetable sous boîtier selon la revendication 1, dans lequel ledit au moins un bras (6) s'étend perpendiculairement depuis ladite face arrière (23).

3. Capteur de pression jetable sous boîtier selon la revendication 1, comprenant en outre au moins une fenêtre ou un orifice, formé en tant que partie intégrante dans ledit seul substrat, pour transmettre une pression de fluide audit au moins un élément de détection.

4. Capteur de pression jetable sous boîtier selon la revendication 3, comprenant en outre au moins une surface d'interface d'étanchéité ou un raccord, intégré dans ledit seul substrat (3), pour rendre étanche ladite fenêtre ou ledit orifice du boîtier par rapport à au moins un orifice de fluide correspondant d'un dispositif.

5. Capteur de pression jetable sous boîtier selon la revendication 1, dans lequel ledit au moins un connecteur électrique (30) et lesdites interconnexions électriques comportent un matériau conducteur déposé sur ledit seul substrat (3).

6. Capteur de pression jetable sous boîtier selon la revendication 1, comprenant des parties de fixation pour recevoir une pluralité desdits éléments de détection, et des interconnexions électriques pour connecter électriquement lesdits éléments de détection auxdits connecteurs électriques respectifs (30).

7. Capteur de pression jetable sous boîtier selon la revendication 1, dans lequel ledit seul substrat (3) comprend un substrat en plastique et dans lequel ledit substrat en plastique est métallisé de façon à former une grille de connexion en plastique ayant ledit au moins un connecteur électrique (30) sur elle.

8. Capteur de pression jetable sous boîtier selon la revendication 1, dans lequel ledit au moins un connecteur électrique (30) comprend des bornes électriques formées sur ledit substrat pour contacter des contacts électriques d'un appareil de mesure pour mesurer les signaux de sortie dudit au moins un élément de détection, lesdites bornes électriques étant connectées électriquement audit au moins un élément de détection.

9. Capteur de pression jetable sous boîtier selon la revendication 1, comprenant en outre au moins un module porteur de fluide, ledit seul substrat étant formé en tant que partie intégrante dudit module porteur de fluide.

10. Capteur de pression jetable sous boîtier selon la revendication 9, dans lequel ledit module porteur de fluide comprend un tube d'écoulement de cathéter jetable.

11. Capteur de pression jetable sous boîtier selon la revendication 1, comprenant en outre une cartouche jetable pour une machine de dialyse et dans lequel au moins un connecteur électrique (30) comprend un raccord mécanique configuré de façon à coopérer avec un raccord correspondant sur ladite cartouche.

12. Capteur de pression jetable sous boîtier selon la revendication 1, comprenant en outre un composant d'ajustage sur ledit substrat (3) connecté électriquement audit au moins un élément de détection pour régler ou décaler le signal de sortie dudit au moins un élément de détection.

13. Capteur de pression jetable sous boîtier selon la revendication 1, dans lequel ledit élément de détection comprend une pastille ou une puce de capteur montée sur ledit seul substrat (3), et comprenant en outre au moins une résistance d'ajustage incorporée déposée sur ladite puce du capteur, ladite résistance d'ajustage incorporée pouvant être ajustée, permettant ainsi la compensation de la portée et/ou du décalage.

14. Procédé de formation d'un capteur de pression jetable sous boîtier sur un seul substrat (3), comprenant une face arrière (23) et au moins un bras (6) qui s'étend vers l'extérieur depuis ladite face arrière dudit substrat, ledit procédé comprenant :
la formation d'interconnexions électriques sur ladite face arrière ;
la fixation d'au moins un élément de détection de pression sur ladite face arrière ;
la soudure de fils pour connecter ledit élément de détection de pression auxdites interconnexions électriques ; et
la formation d'au moins un connecteur électrique (30) sur ledit bras, ledit connecteur électrique fournissant une connexion auxdites interconnexions électriques de manière à ce que les signaux de sortie venant dudit au moins un élément de détection de pression puissent être couplés à un appareil externe.

15. Procédé selon la revendication 14, dans lequel ledit seul substrat (3) comprend un substrat en plastique, comprenant en outre la formation dudit substrat en plastique en utilisant un procédé de moulage, ledit substrat en plastique étant un substrat en plastique moulé.

16. Procédé selon la revendication 15, dans lequel ladite formation desdites interconnexions électriques et ladite formation du connecteur électrique (30) comprennent le dépôt d'un matériau conducteur sur ledit substrat en plastique.
